# EUROPEAN PATENT APPLICATION

(11) **EP 3 070 157 A1**
(43) Date of publication of application: **21.09.2016**
(21) Application number: 14859715.6
(22) Date of filing: 10.10.2014
(51) Int. Cl.: C12M 1/42, C12M 1/34

(54) **CELL OBSERVATION APPARATUS**

(30) Priority: 11.11.2013 JP 2013233014
(71) Applicant: Hamamatsu Photonics K.K., Hamamatsu-shi, Shizuoka 435-8558 (JP)
(72) Inventor: ITO Taira, Hamamatsu-shi Shizuoka 435-8558 (JP); TAKAHASHI Akira, Hamamatsu-shi Shizuoka 435-8558 (JP)
(74) Representative: Hoffmann Eitle
(86) International application number: PCT/JP2014/077250
(87) International publication number: WO 2015/068530

(57) **Abstract**

A cell observation apparatus is a cell observation apparatus for observing a cell held by a microplate having an array of wells holding respective samples, which comprises a microplate holder for the microplate to be mounted thereon, and an electrical stimulation unit in which a plurality of electrode pairs, each electrode pair including a negative electrode and a positive electrode, are arranged, wherein the negative electrode and the positive electrode are coated with respective insulators except for their leading ends facing the well, and the leading ends form respective electrode portions exposed to the outside, and wherein a space penetrating upward from the electrode portions is formed between the negative electrode and the positive electrode.

## Description

### Technical Field

The present invention relates to a cell observation apparatus for observing a reaction of a sample including a cell as an object, to electrical stimulation applied thereto.

### Background Art

In the field of drug discovery screening, influence of a drug administered to a sample of cells or the like is evaluated by measuring light emitted from the cells in certain cases. On that occasion, for controlling a membrane potential of the cells or for giving a role of a pacemaker for myocardial cells, it is common practice to apply electrical stimulation to the cells. Particularly, in the usage of pacing of myocardial cells, an arrhythmic situation can be created and, for this reason, there is a growing need for measurement using the electrical stimulation, with the recent spread of drug discovery studies by making use of stem cells.

In the drug discovery screening, a microplate having an array of wells for cells to be placed therein is used for collectively evaluating a plurality of drugs. Patent Literature 1 discloses a measurement device which monitors a biological response to electric field stimulation of the cells placed in the wells of the microplate, by fluorescence detection. This measurement device employs a configuration in which an electrode pair of a flat plate shape or a coaxial shape consisting of a positive electrode and a negative electrode can be located in each of the wells wherein the cells are placed. This electrode pair has a structure in which an insulator is embedded in a gap between two electric conductors as the positive electrode and negative electrode.

### Citation List

### Patent Literature

Patent Literature 1: Japanese Unexamined Patent Publication No. 2005-514909

### Summary of Invention

### Technical Problem

In performing the measurement with use of the measurement device described in the above-mentioned Patent Literature 1, the electrode pair is inserted into the well in which the cells as a measurement object and a solution are placed. On that occasion, since the insulator is embedded in between the electrode pair, bubbles generated at the tip of the electrode pair or gas generated from the cells tends to stay within the well. Furthermore, heat generated with application of the electrical stimulation by the electrode pair also tends to accumulate within the well. Influence of such bubbles, gas, or heat made accurate observation of light from the cells difficult in some cases.

The present invention has been accomplished in view of the above-mentioned problem and it is an object of the present invention to provide a cell observation apparatus capable of improving accuracy of cell observation with application of electrical stimulation.

### Solution to Problem

For solving the above-mentioned problem, a cell observation apparatus in accordance with one aspect of the present invention is a cell observation apparatus for observing a cell held by a sample case having an array of holding units holding a sample including the cell; the cell observation apparatus comprises a mounting unit for the sample case to be mounted thereon, and an electrical stimulation unit in which a plurality of electrode pairs, each electrode pair including a first electrode and a second electrode, are arranged; at least one of the first electrode and the second electrode is coated with an insulating material except for a leading end thereof facing the holding unit, and the leading end forms an electrode portion exposed to the outside; a space penetrating upward from the electrode portion is formed between the first electrode and the second electrode.

In the cell observation apparatus as described above, the electrode pair including the first electrode and the second electrode is placed in each of the holding units arrayed in the sample case, whereby this electrode pair can apply electrical stimulation by electric currents to the sample including the cell immersed in a solution. Since the space penetrating upward from the electrode portion exposed to the outside at the leading end of either one electrode is formed between the first electrode and the second electrode constituting the electrode pair, a region around the electrode portion between the first electrode and the second electrode is filled with the solution on the occasion of insertion of the electrode pair into the holding unit. This allows bubbles generated at the tip of the electrode pair or gas generated from the cell to be discharged through the space between the electrodes to the outside of the holding unit and also allows heat generated in the holding unit with application of the electrical stimulation to be dissipated through the solution between the electrodes to the outside of the holding unit. As a consequence, the reaction of the cell to the electrical stimulation can be accurately observed without influence of bubbles, gas, or heat.

### Advantageous Effects of Invention

The present invention can achieve improvement in accuracy of cell observation with application of electrical stimulation.

### Brief Description of Drawings

Fig. 1 is a drawing showing a schematic configuration of a cell observation apparatus 1 in accordance with a preferred embodiment of the present invention.
Fig. 2 is a perspective view showing a configuration of a microplate 20 in Fig. 1.
Fig. 3 is a side sectional view showing a cross-sectional structure of the microplate 20 in Fig. 1.
Fig. 4A is a vertical sectional view along an insertion direction of an electrode pair 17 in an inserted situation of an electrical stimulation unit 16 in Fig. 1 with respect to a well 21, and Fig. 4B is a plan view of the electrode pair 17 in Fig. 1 viewed from a bottom face 22 side of the well 21.
Fig. 5A is a vertical sectional view along an insertion direction of an electrode pair 107 in accordance with a modification example of the present invention, and Fig. 5B is a plan view of the electrode pair 107 viewed from the bottom face 22 side of the well 21.
Fig. 6 is waveform charts showing the results of observation of cells with use of the cell observation apparatus 1 having the electrode pairs 107 in Fig. 5A.
Fig. 7 is waveform charts showing the results of observation of cells with use of the cell observation apparatus 1 having the electrode pairs 107 in Fig. 5A.
Fig. 8 is waveform charts showing the results of observation of cells with use of the cell observation apparatus 1 having the electrode pairs 107 in Fig. 5A.
Fig. 9 is waveform charts showing the results of observation of cells with use of the cell observation apparatus 1 having the electrode pairs 107 in Fig. 5A.
Fig. 10A is a vertical sectional view along an insertion direction of an electrode pair 117 in accordance with another modification example of the present invention, and Fig. 10B is a plan view of the electrode pair 117 viewed from the bottom face 22 side of the well 21.
Fig. 11A is a vertical sectional view along an insertion direction of an electrode pair 127 in accordance with another modification example of the present invention, and Fig. 11B is a plan view of the electrode pair 127 viewed from the bottom face 22 side of the well 21.
Figs. 12A to 12C are perspective views showing structures of electrode pairs in accordance with other modification examples of the present invention.
Figs. 13A to 13B are perspective views showing structures of electrode pairs in accordance with other modification examples of the present invention.
Figs. 14A to 14F are perspective views showing structures of electrode pairs in accordance with other modification examples of the present invention.

### Description of Embodiments

Embodiments of the cell observation apparatus according to the present invention will be described below in detail with reference to the accompanying drawings. In the description of the drawings the same elements will be denoted by the same reference signs, without redundant description. It should be noted that each of the drawings was prepared for purpose of description and made with special emphasis on objects of description. For this reason, dimensional ratios of each member in the drawings do not always agree with actual ones.

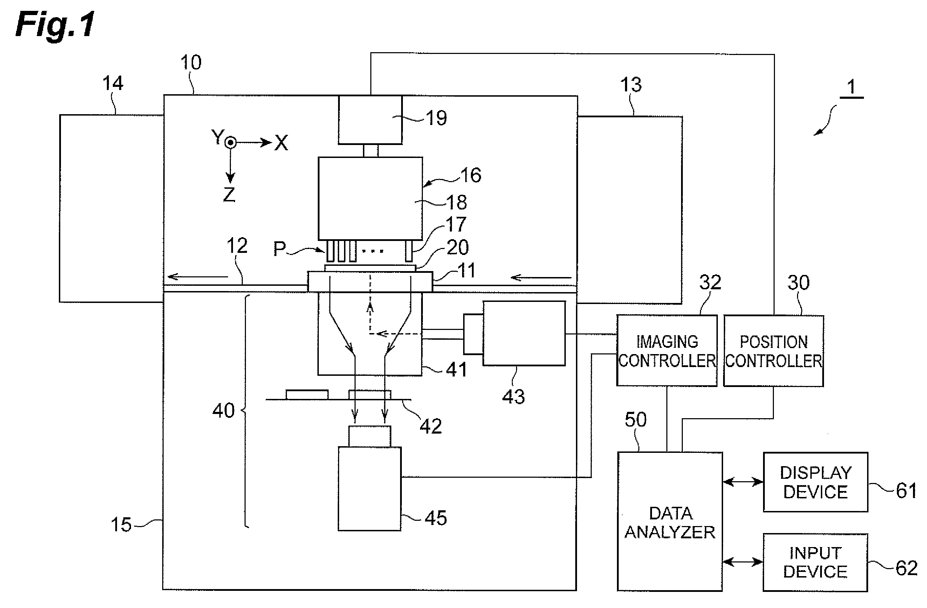

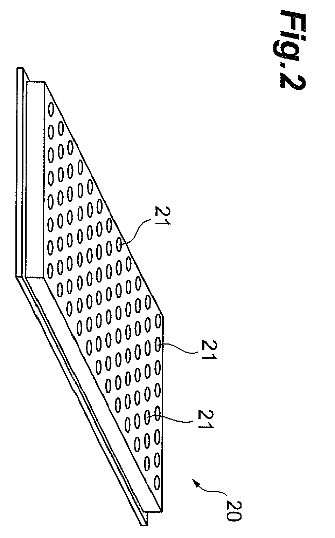

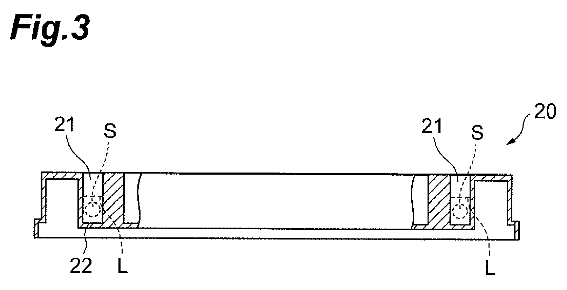

Fig. 1 is a configuration diagram schematically showing one embodiment of the cell observation apparatus 1 according to the present invention. Fig. 2 is a perspective view showing one example of a configuration of a microplate 20. Fig. 3 is a side sectional view showing a cross-sectional structure of the microplate 20 shown in Fig. 2. The cell observation apparatus 1 of the present embodiment is a device that uses the microplate 20 as a sample case and that is provided for measuring fluorescence from samples S placed at a measurement position P while being held by the microplate 20.

The sample S includes a predetermined cell or predetermined cells. Examples of the predetermined cells include myocardial cells, neurons, and so on. The cell observation apparatus of the present embodiment cannot be applied only to the fluorescence measurement but can also be generally applied to light measurement to measure light emitted from samples, e.g., phosphorescence and luminescence. The following will describe the configuration of the cell observation apparatus 1.

The cell observation apparatus 1 shown in Fig. 1 is configured with a data acquisition device 10, a position controller 30, an imaging controller 32, and a data analyzer 50. The data acquisition device 10 has a dark box 15 inside which the microplate 20 holding the cells as objects of fluorescence observation is contained, and a moving image acquisition unit 40 which is installed inside the dark box 15 and which is used for measurement of fluorescence from the samples S placed at the measurement position P.

As shown in Figs. 2 and 3, the microplate 20 used as the sample case in the present embodiment is a plate-like member in which a plurality of wells (holding units) 21 are arranged in a two-dimensional array, and is configured so that the sample S, together with a solution L such as a culture solution, can be held in each of the plurality of wells 21. In the configuration example shown in these figures, the plurality of wells 21 are arranged in the form of the two-dimensional array of 8 × 12 = 96 circular wells 21 and each well 21 holds the sample S as immersed in the solution L, in the vicinity of a bottom face 22 thereof. The plurality of wells 21 may be an array of an arbitrary number of wells 21, e.g., 384 wells. Examples of cross-sectional shape of the well 21 include a circle, an ellipse, a rectangle, and so on. The bottom face 22 of this microplate 20 is formed of a material which can transmit excitation light for fluorescence measurement applied to the samples S and the fluorescence emitted from the samples S. In general, it is sufficient in the cell observation apparatus 1 that the bottom face 22 of the microplate 20 be formed of a material which can transmit light emitted from the samples S as measurement objects.

Referring back to Fig. 1, the microplate 20 is mounted on a microplate holder (mounting unit) 11 having an opening for observation of fluorescence, in the dark box 15. A microplate transfer mechanism 12 for transferring these microplate 20 and microplate holder 11 in a predetermined direction (a direction from the right side to the left side in Fig. 1) in the dark box 15 is also installed in the dark box 15.

On one side of the dark box 15 serving as an inlet side in the transfer direction of the microplate 20 in the transfer mechanism 12, there is an inlet-side microplate stocker 13 installed for stocking a predetermined number of (e.g., 25) microplates 20 before measurement with the samples S and solutions L held therein. On the other side of the dark box 15 serving as an outlet side in the transfer direction of the microplate 20, there is an outlet-side microplate stocker 14 installed for stocking the microplates 20 after measurement.

In this configuration, the microplate 20 carried from the inlet-side microplate stocker 13 into the dark box 15 is held by the microplate holder 11 and transferred by the transfer mechanism 12. The microplate 20 is once stopped at the measurement position P and, in this state, necessary light measurement is carried out for the samples S held by the microplate 20. After completion of the measurement, the microplate 20 is again transferred by the transfer mechanism 12, so as to be discharged into the outlet-side microplate stocker 14. It is noted that in Fig. 1 the transfer mechanism 12 and the stockers 13, 14 are depicted without illustration of specific configurations for carrying in, transferring, and discharging the microplate 20.

Above the measurement position P where the microplate 20 and samples S are placed during execution of the fluorescence measurement, there is an electrical stimulation unit 16 installed for being inserted into the wells 21 of the microplate 20 and applying electrical stimulation by electric currents to the samples S immersed in the solutions L. Below the measurement position P, the moving image acquisition unit 40 is installed for being used for detection of fluorescence emitted through the bottom face 22 of the microplate 20 from the samples S contained in the wells 21.

The moving image acquisition unit 40 is a moving image acquisition means which detects a two-dimensional optical image representing a two-dimensional optical intensity distribution of the microplate 20 including light emitted from the samples S held in the wells 21 of the microplate 20 and which acquires moving image data of the two-dimensional optical image. The two-dimensional optical image to be detected may be an optical intensity distribution including light emitted from the sample S held in at least one well 21. This moving image acquisition unit 40 is constituted by an imaging device 45, a light-guiding optical system 41, an optical filter unit 42, and an excitation light source 43. The imaging device 45 has a two-dimensional pixel structure in which a plurality of pixels are two-dimensionally arranged, and detects a fluorescence image which is a two-dimensional light detection image of the fluorescence emitted from the sample S. The imaging device 45 to be used herein can be, for example, a highly-sensitive CCD camera or CMOS imaging camera. An image intensifier, a relay lens, and others may be placed in front of the camera, if necessary, so as to construct the moving image acquisition unit 40. The moving image acquisition unit 40 may be configured to acquire still images, with a function as an image acquisition unit to acquire moving images and/or still images.

The light-guiding optical system 41 is installed between the measurement position P where the microplate 20 is placed, and the imaging device 45. The light-guiding optical system 41 is an optical system which guides the two-dimensional optical image as a view from the bottom face 22 of the microplate 20 with the samples S and solutions L held in the respective wells 21, to the imaging device 45. A specific configuration of the light-guiding optical system 41 may be suitably constructed by optical elements which can exercise necessary functions (e.g., a light condensing function, an optical image reducing function, and so on), depending upon the configurations of the microplate 20 and imaging device 45 and others. An example of such optical elements is a tapered fiber (cf. Japanese Unexamined Patent Publication No. 2001-188044). The light-guiding optical system 41 may also be configured using a light irradiation device with a light-guiding member having concavities and convexities (cf. Japanese Unexamined Patent Publication Nos. 2010-230397 and 2010-230396).

In Fig. 1, the optical filter unit 42, which can implement placement of an optical filter onto a light-guiding optical path of fluorescence, switching of optical filters, and so on according to needs, is further installed between the light-guiding optical system 41 and the imaging device 45. The optical filter unit 42 of this configuration is used for cutting light at the wavelength of the excitation light emitted from the back face of the light-guiding optical system 41. However, such optical filter unit 42 may be excluded if unnecessary.

The excitation light source 43 is an excitation light supply means for supplying the excitation light for fluorescence measurement to the samples S. A specific configuration of the excitation light source 43 may be suitably constructed depending upon types of samples S as objects of fluorescence measurement, the wavelength of the excitation light applied to the samples S, and so on, but, for example, it can be constituted by an illumination light source such as LED for supplying light, and an optical filter unit for implementing selection and switching of the wavelength of the excitation light. The apparatus may be configured without the excitation light source 43 if the supply of the excitation light is unneeded, depending upon types of light measurement performed with the samples S.

In the present embodiment, the light-guiding optical system 41 is constructed as an optical system which can guide the two-dimensional optical image from the microplate 20 and samples S to the imaging device 45 and guide the excitation light from the excitation light source 43 to the samples S. The optical system of this kind can be configured, for example, using a dichroic mirror which transmits the fluorescence from the microplate 20 and reflects the excitation light from the excitation light source 43, or the like. The light-guiding optical system 41 may be configured of a light-guiding member with concavities or convexities in its top face, as described in Japanese Unexamined Patent Publication Nos. 2010-230397 and 2010-230396, or may be configured by coating the top face with a light exit medium such as water or oil. In Fig. 1, optical paths of the fluorescence and excitation light in the light-guiding optical system 41 are schematically shown by solid lines and broken lines, respectively.

The configuration of the electrical stimulation unit 16 will be described below in detail. The electrical stimulation unit 16 has a structure in which a plurality of electrode pairs 17 extending vertically toward the microplate 20 are secured to a base part 18 so as to be two-dimensionally arranged. Specifically, the electrode pairs 17 are two-dimensionally arranged corresponding to the two-dimensional array arrangement of the plurality of wells 21 of the microplate 20, while extending so as to face the wells 21 of the microplate 20.

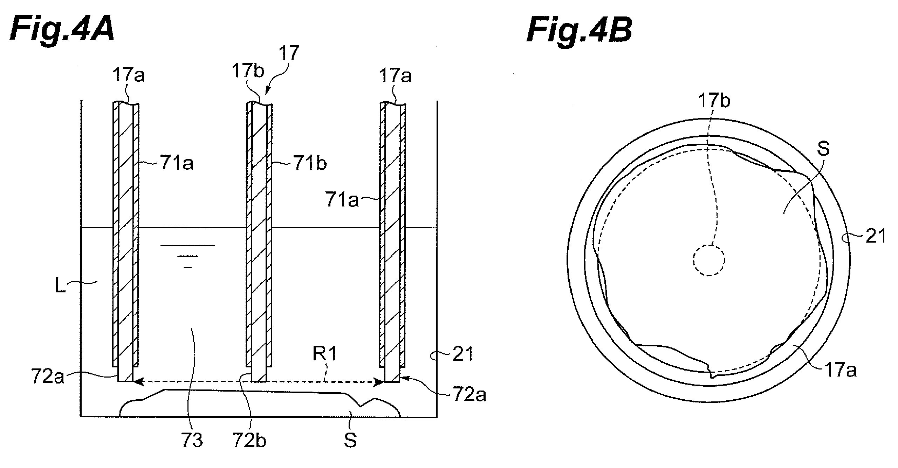

Figs. 4A-4B show a structure of each of the electrode pairs 17 constituting the electrical stimulation unit 16, wherein Fig. 4A is a vertical sectional view along the insertion direction of the electrode pair 17 in an inserted situation of the electrical stimulation unit 16 with respect to the well 21 and Fig. 4B is a plan view of the electrode pair 17 viewed from the bottom face 22 side of the well 21.

As shown in the these figures, each electrode pair 17 is composed of a negative electrode (second electrode) 17a of such a cylindrical shape as to open at a leading end thereof and surround a positive electrode 17b, and the positive electrode (first electrode) 17b of a rod shape (e.g., a circular column shape) inserted inside the negative electrode 17a so as to be placed on and along the center axis of the negative electrode 17a, while the negative electrode 17a has an outer diameter smaller than an inner diameter of the well 21. The electrode pair 17 also has such a structure that the leading end of the negative electrode 17a and a leading end of the positive electrode 17b are located at the same height from the bottom face of the well 21 to them. Namely, the negative electrode 17a and positive electrode 17b form a coaxial shape. Furthermore, these negative electrode 17a and positive electrode 17b are provided with a coating of platinum for imparting wettability to the inner and outer faces thereof.

The cylindrical shape of the negative electrode 17a may be circular or elliptical in cross section. This allows the rod-shaped positive electrode 17b to be contained within the negative electrode 17a of the cylindrical shape. The electrode pair 17 does not always have to be limited to the configuration wherein each of the negative electrode 17a and positive electrode 17b is composed of one member, but it may have a configuration wherein either one or both of them are composed of a plurality of members.

Furthermore, the inner face and outer face of the negative electrode 17a are coated with an insulator (insulating material) 71a except for the leading end thereof facing the sample S when inserted. In this configuration, the negative electrode 17a comes to have an electrode portion 72a exposed to the outside at its leading end. Similarly, the side face of the positive electrode 17b is coated with an insulator (insulating material) 71b except for the leading end thereof facing the sample S when inserted. In this configuration, the positive electrode 17b comes to have an electrode portion 72b exposed to the outside at its leading end. These insulators 71a, 71b are formed by use of insulating shrinkable tubes or by coating with an insulating substance.

The surfaces of the insulators 71a, 71b may be provided with wettability, at least in part thereof on the electrode portion 72a, 72b side. For example, the insulators 71a, 71b themselves may be made of a material with wettability (e.g., hydrophilic silicone rubber or the like), or the insulators 71a, 71b may be partly coated with a material with wettability (e.g., platinum, a hydrophilic ceramic, or the like).

In the electrode pair 17 of the structure as described above, a space 73 penetrating upward from the electrode portions 72a, 72b to above the well 21 is formed between the negative electrode 17a and positive electrode 17b and, the negative electrode 17a and positive electrode 17b are provided with wettability. This configuration allows the solution L to penetrate from the electrode portions 72a, 72b into the space 73, on the occasion of insertion of the electrode pair 17 into the well 21.

Referring back to Fig. 1 again, the electrical stimulation unit 16 is provided with a shifter mechanism 19 for supporting the electrode pairs 17 through the base part 18. The shifter mechanism 19 is a driving mechanism for moving the electrode pairs 17 toward or away from the microplate 20 (along the Z-direction in Fig. 1) and also moving the electrode pairs 17 in directions along the bottom face 22 of the microplate 20 (or in directions along a plane including the X- and Y-axes in Fig. 1), which performs such driving that the electrode pairs 17 are placed in the respective opposed wells 21 in observation of the samples S and that the electrode pairs 17 are retracted from within the wells 21 after completion of observation of the samples S.

Connected to the data acquisition device 10 of this configuration are the position controller 30 and the imaging controller 32. The position controller 30 is electrically connected to the shifter mechanism 19 and controls the shifter mechanism 19 so as to place the electrode pairs 17 in the wells 21 of the microplate 20 at a start of light measurement of the samples S. The position controller 30 is also electrically connected to the electrode pairs 17 and applies respective voltages to the negative electrodes 17a and positive electrodes 17b so as to make a potential difference between the negative electrodes 17a and positive electrodes 17b of the electrode pairs 17. The imaging controller 32 controls application of the excitation light by the excitation light source 43 and imaging of the two-dimensional fluorescence image in the microplate 20 by the imaging device 45.

Furthermore, the data analyzer 50 is connected to the position controller 30 and the imaging controller 32. This data analyzer 50 is an analysis processing means which acquires the moving image data including the light detection image acquired by the moving image acquisition unit 40, via the imaging controller 32 and which performs analysis processing on an object of the moving image data. The data analyzer 50 also controls the operations of the respective parts of the data acquisition device 10 via the position controller 30 and the imaging controller 32, thereby controlling the fluorescence measurement for the samples S in the cell observation apparatus 1. Further connected to the data analyzer 50 are a display device 61 for displaying the measurement result and others and an input device 62 used for input of data, input of instructions necessary for the fluorescence measurement, and so on.

In the cell observation apparatus 1 as described above, the electrode pairs 17 including the negative electrodes 17a and positive electrodes 17b are placed in the wells 21 arrayed in the microplate 20, whereby the electrode pairs 17 apply the electrical stimulation by electric currents to the samples S immersed in the solutions L. In this regard, since the space 73 penetrating upward from the electrode portions 72a, 72b exposed to the outside at the leading ends is formed between the negative electrode 17a and positive electrode 17b constituting each electrode pair 17, the region around the electrode portions 72a, 72b between the negative electrode 17a and positive electrode 17b is filled with the solution L on the occasion of insertion of the electrode pair 17 into the well 21. This allows bubbles generated at the tip of the electrode pair 17 or gas generated from the cells to be discharged to the outside of the well 21 through the space between the electrodes and also allows heat generated in the well 21 with application of electrical stimulation to be dissipated to the outside of the well 21 through the solution L between the electrodes. As a consequence, apparatus 1 can accurately observe the reaction of the sample S such as a cell to the electrical stimulation, without influence of the bubbles, gas, or heat

Since the coated portions of the negative electrode 17a and positive electrode 17b with the insulators 71a, 71b are provided with wettability at least in part thereof, the solution L can be efficiently drawn into the space 73 between the negative electrode 17a and positive electrode 17b on the occasion of insertion of the electrode pair 17 into the well 21, and thus apparatus 1 can more accurately observe the reaction of the sample S such as a cell.

Since the electrode pair 17 has the coaxial structure and is coated with the insulators 71a, 71b except for the leading ends, paths R1 (cf. Fig. 4A) of electric currents made to flow in the solution L in the well 21 by the electrode pair 17 can be concentrated near the electrode portions 72a, 72b. As a result, the current density of electric currents supplied to the sample S can be efficiently increased, whereby great electrical stimulation can be applied to the cell with low power consumption. In this case as well, the current density can be kept at a sufficient level neat the sample S and thus it is feasible to fully achieve pacing of myocardial cells and control on opening/closing of ion channels of cells. Since a current-flowing region is restricted, the voltage supplied to the electrode pair 17 is kept lower, so as to reduce power consumption. The effect of reduction in power consumption is greater, particularly, in the case where the microplate 20 to be used is one having the structure with a large number of wells 21 arrayed, e.g., 384 wells.

The present invention is not limited to the above-mentioned embodiment.

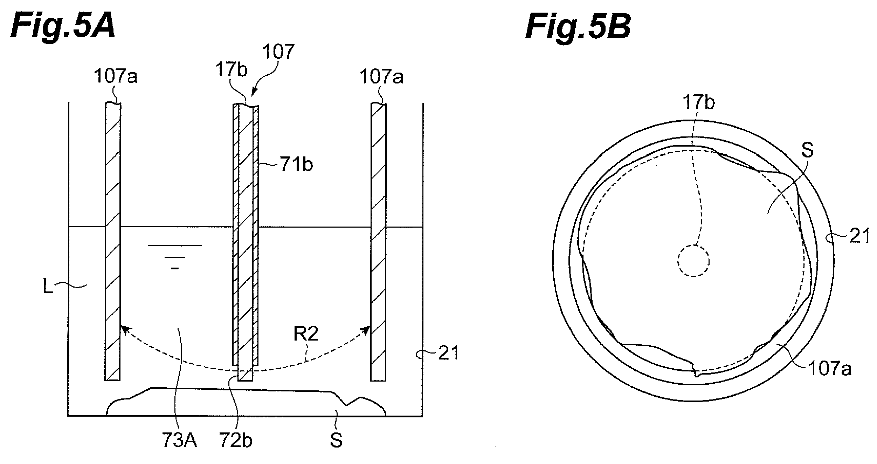

For example, the structure of the electrode pair 17 in the electrical stimulation unit 16 is not limited to the coaxial shape but can employ various shapes. For example, Figs. 5A-5B show a structure of an electrode pair 107 in accordance with a modification example of the present invention, wherein Fig. 5A is a vertical sectional view along the insertion direction of the electrode pair 107 in an inserted state thereof in the well 21 and Fig. 5B is a plan view of the electrode pair 107 viewed from the bottom face 22 side of the well 21. As shown in these figures, the electrode pair 107 is different from the electrode pair 17, in that the electrode pair 107 has a negative electrode 107a of a cylindrical shape entirely exposed to the outside without any coating of insulator, instead of the negative electrode 17a coated with the insulator 71a. The electrode pair 107 of this structure can also concentrate paths R2 of electric currents made to flow through the solution L in the well 21, near the electrode portion 72b close to the central region of the bottom face of the well 21. Since a space 73A penetrating upward is formed between the leading end of the positive electrode 17b and the negative electrode 107a, the space 73A can be filled with the solution L on the occasion of insertion of the electrode pair 107 into the well 21.

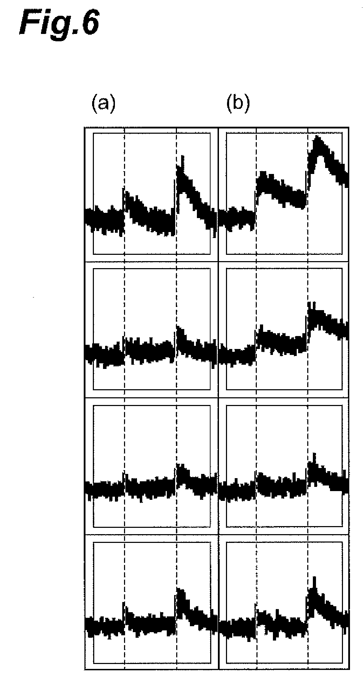

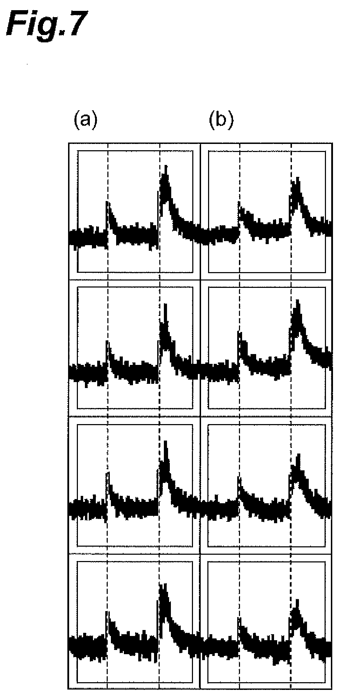

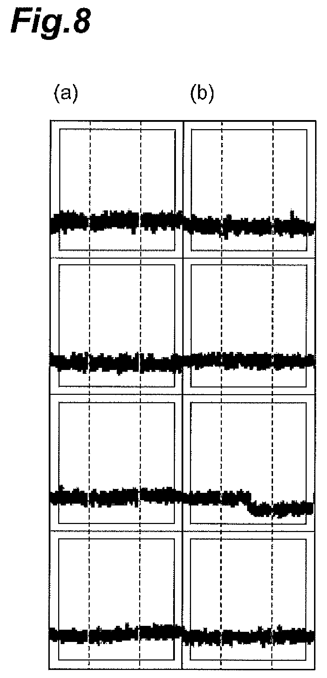

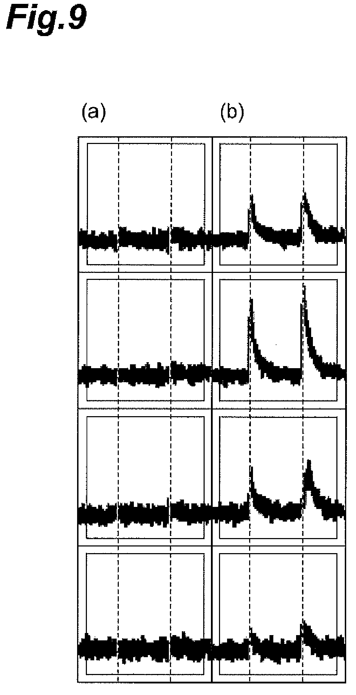

Figs. 6 to 9 show the results of observation of cells with use of the cell observation apparatus 1 having the electrode pairs 107 exposed in the length of 1 mm at the leading end of the positive electrode 17b. However, each electrode pair 107 in this case has a structure in which a leading end of the negative electrode 107a is more projecting toward the bottom face of the well 21 than the leading end of the positive electrode 17b. Fig. 6 is waveform charts showing temporal changes of optical intensity observed in a plurality of wells 21, each of which was obtained with application of the applied voltage of 7 V to the electrode pair 107 and with an object of sample S immersed in the solution (culture solution) of 100 µl in the well 21; Fig. 7 is waveform charts showing temporal changes of optical intensity each of which was obtained with application of the applied voltage of 7 V and with an object of sample S immersed in the solution of 200 µl; Fig. 8 is waveform charts showing temporal changes of optical intensity each of which was obtained with application of the applied voltage of 2 V and with an object of sample S immersed in the solution of 100 µl; Fig. 9 is waveform charts showing temporal changes of optical intensity each of which was obtained with application of the applied voltage of 2 V and with an object of sample S immersed in the solution of 200 µl; in each of the figures, (a) shows the measurement results in the configuration wherein the positive electrodes 17b are not coated with the insulator 71b and (b) shows the measurement results in the configuration wherein the positive electrodes 17b are coated with the insulator 71b. The measurement results of Figs. 7 and 9 show the cases with the large amount of the solution L where the liquid level of the solution L is high in the wells 21.

As shown in these measurement results, in the case of the applied voltage of 7 V, the reaction to the electrical stimulation was obtained under all the conditions, whereas, with decrease of the applied voltage to 2 V, the reaction was obtained only in the case where the insulator 71b was provided and where the liquid level of the solution L was high in the wells 21. It is seen from this result that the reaction of the cell to electrical stimulation appears even with the low voltage applied to the electrodes, in the structure of the electrode coated with the insulator except for the leading end part and provided with the upwardly-penetrating space for the solution L formed between the electrodes. The reaction appears even with decrease of the voltage applied to the electrode pair, which means that the structure is suitable for power saving.

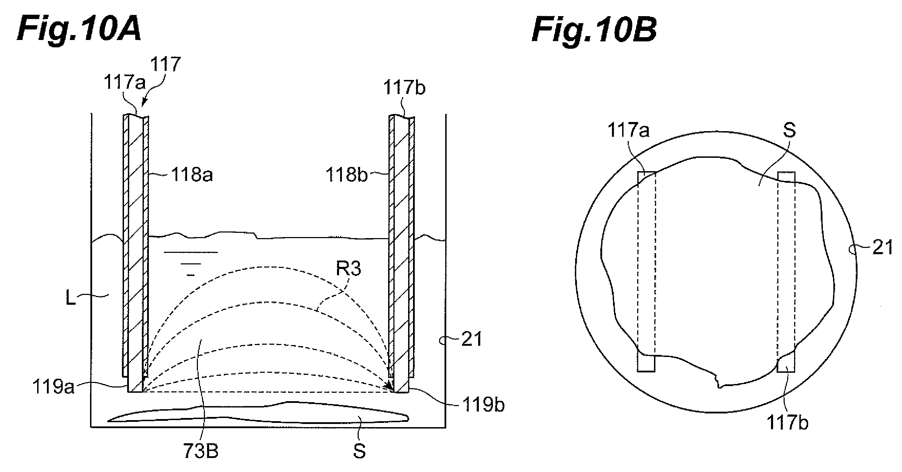

Figs. 10A-10B show a structure of an electrode pair 117 in accordance with another modification example of the present invention, wherein Fig. 10A is a vertical sectional view along the insertion direction of the electrode pair 117 in an inserted state thereof in the well 21 and Fig. 10B is a plan view of the electrode pair 117 viewed from the bottom face 22 side of the well 21. As shown in these figures, the electrode pair 117 has a pair of positive electrode 117a and negative electrode 117b of a flat plate shape extending toward the bottom face of the well 21, and both faces thereof are coated with insulators 118a, 118b, respectively, except for electrode portions 119a, 119b which are leading ends facing the bottom face of the well 21. These positive electrode 117a and negative electrode 117b are arranged opposite to each other so as to be parallel to each other. The electrode pair 117 of this configuration can also concentrate paths R3 of electric currents made to flow through the solution L in the well 21, in the vicinity of the electrode portions 119a, 119b near the bottom face of the well 21. Since an upwardly-penetrating space 73B is formed between the leading end of the positive electrode 117a and the negative electrode 117b, the space 73B can be filled with the solution L on the occasion of insertion of the electrode pair 117 into the well 21.

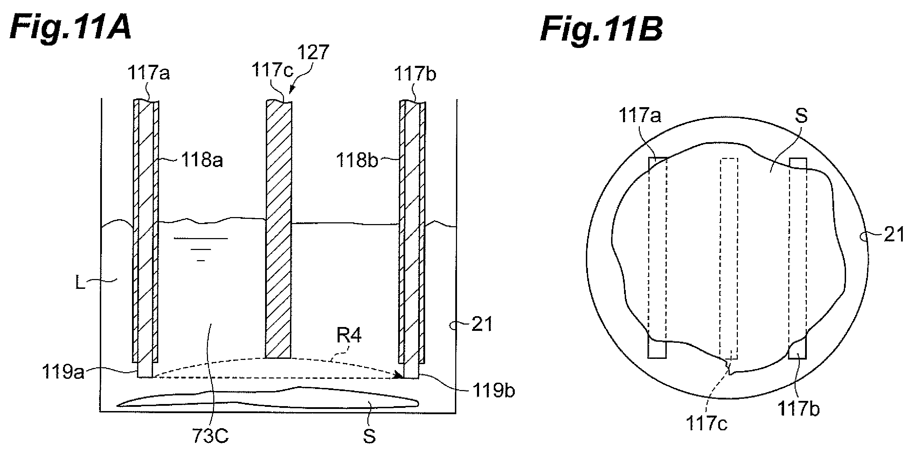

Figs. 11A-11B show a structure of an electrode pair 127 in accordance with another modification example of the present invention, wherein Fig. 11A is a vertical sectional view along the insertion direction of the electrode pair 127 in an inserted state thereof in the well 21 and Fig. 11B is a plan view of the electrode pair 127 viewed from the bottom face 22 side of the well 21. As shown in the same figure, the electrode pair 127 has an insulating plate 117c on a flat plate extending in parallel and opposite to the positive electrode 117a and negative electrode 117b, halfway between the positive electrode 117a and negative electrode 117b of the flat plate shape. The leading ends of the positive electrode 117a and negative electrode 117b are more projecting toward the bottom face of the well 21 than a leading end of the insulating plate 117c. The electrode pair 127 of this configuration can concentrate paths R4 of electric currents made to flow through the solution L in the well 21, between the central region of the bottom face of the well 21 and the regions near the electrode portions 119a, 119b, so as to increase the current density of electric currents supplied to the whole sample S. Since upwardly-penetrating spaces 73C are formed between the leading end of the positive electrode 117a and the insulating plate 117c and between the leading end of the negative electrode 117b and the insulating plate 117c, the spaces 73C can be filled with the solution L on the occasion of insertion of the electrode pair 127 into the well 21.

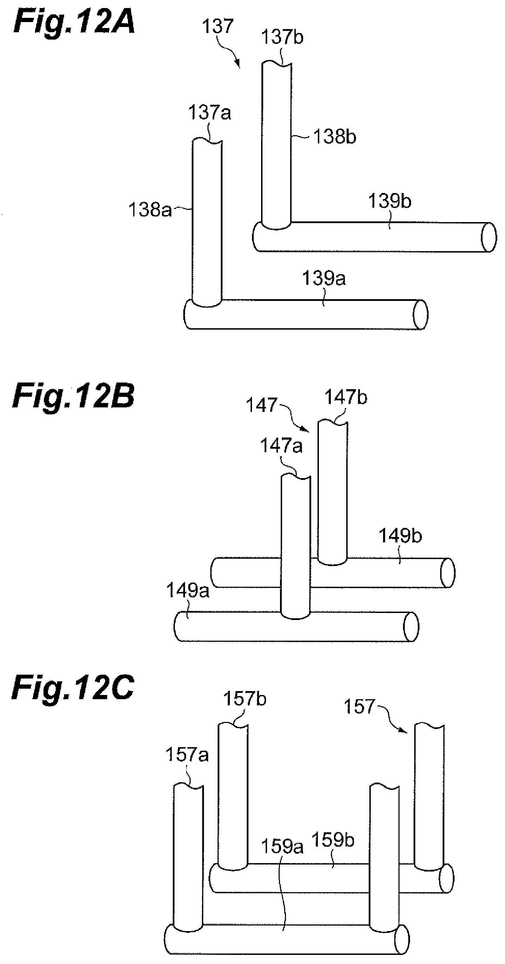

Furthermore, Figs. 12A-12C, 13A-13B, 14A-14F show structures of other modification examples of the electrode pair.

An electrode pair 137 shown in Fig. 12Ahas a positive electrode 137a and a negative electrode 137b of a rod shape extending in parallel to each other toward the bottom face of the well 21 and their leading ends are configured to be bent in an L-shape so as to extend in parallel to each other along the bottom face of the well 21. The positive electrode 137a and negative electrode 137b are coated with respective insulators 138a, 138b except for their leading ends, whereby the leading ends constitute electrode portions 139a, 139b exposed to the outside. An electrode pair 147 shown in Fig. 12B has a positive electrode 147a and a negative electrode 147b of a two-rod configuration, just as the electrode pair 137 does, and their leading ends are configured to be bent in a T-shape so as to extend in parallel to each other along the bottom face of the well 21. In the positive electrode 147a and negative electrode 147b, the leading ends thereof constitute electrode portions 149a, 149b exposed to the outside. Furthermore, an electrode pair 157 shown in Fig. 12C has a positive electrode 157a and a negative electrode 157b each including two rod-shaped electrodes parallel to each other, and their leading ends are configured in a U-shape along the bottom face of the well 21 so as to connect the tips of the two parallel electrodes. Namely, the leading ends of the positive electrode 157a and negative electrode 157b constitute electrode portions 159a, 159b extending in parallel to each other and exposed to the outside. The electrode pairs 137, 147, and 157 of these structures can also efficiently apply the electrical stimulation in a large current density to a wide range of the sample S placed in the well 21.

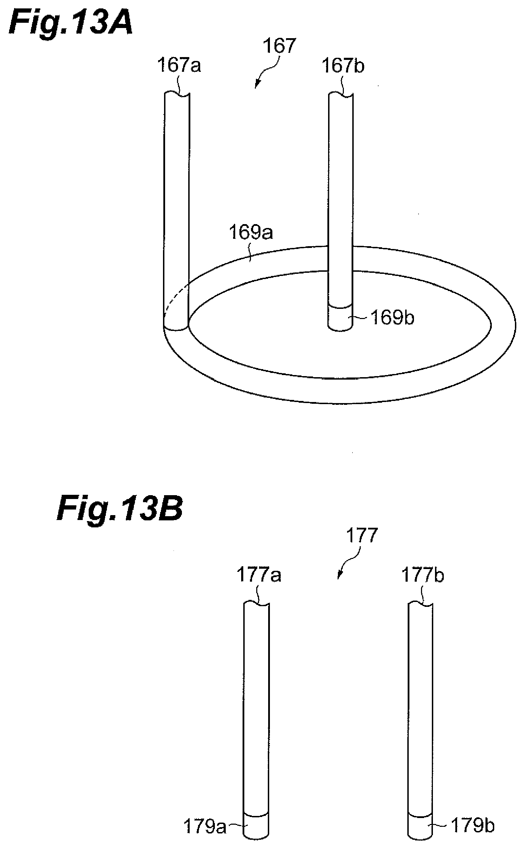

An electrode pair 167 shown in Fig. 13A has a positive electrode 167a and a negative electrode 167b of a rod shape extending in parallel to each other toward the bottom face of the well 21 and a leading end of the positive electrode 167a is configured in a ring shape along the bottom face of the well 21 so that a leading end of the negative electrode 167b is surrounded by the leading end of the positive electrode 167a. In the positive electrode 167a and negative electrode 167b, the leading ends thereof constitute respective electrode portions 169a, 169b exposed to the outside. An electrode pair 177 shown in Fig. 13B has a positive electrode 177a and a negative electrode 177b of a rod shape extending in parallel to each other toward the bottom face of the well 21 and leading ends of the respective rod electrodes constitute electrode portions 179a, 179b exposed to the outside.

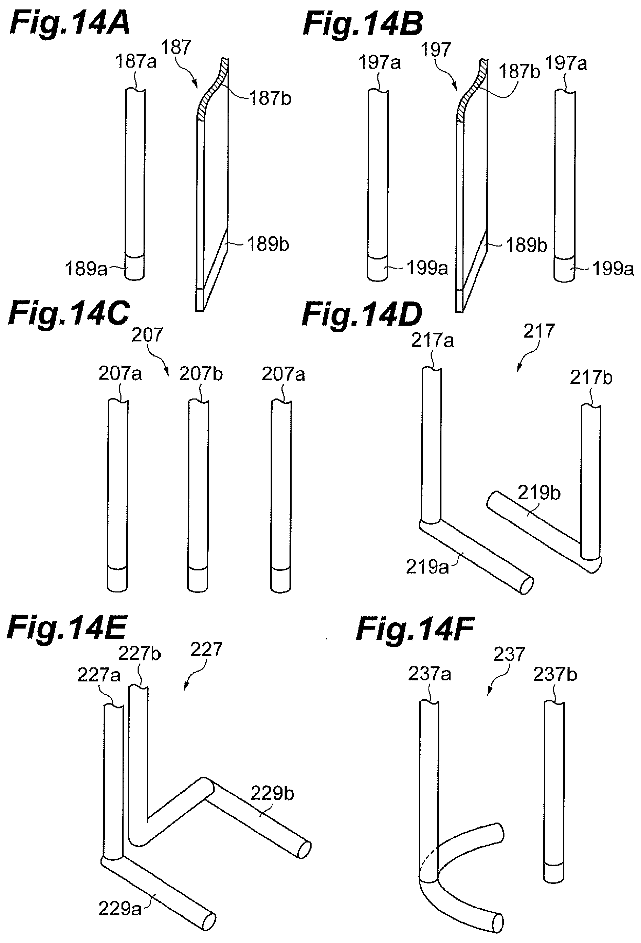

An electrode pair 187 shown in Fig. 14A has a positive electrode 187a of a rod shape and a negative electrode 187b of a flat plate shape extending in parallel to each other toward the bottom face of the well 21 and leading ends of the respective electrodes constitute electrode portions 189a, 189b exposed to the outside. Namely, the positive electrode 187a is arranged along the surface of the negative electrode 187b. Furthermore, an electrode pair 197 shown in Fig. 14B has positive electrodes 197a which are two rod-shaped electrodes extending toward the bottom face of the well 21 so as to interpose the negative electrode 187b in between them on both sides, instead of the positive electrode 187a of the electrode pair 187. Leading ends of the positive electrodes 197a constitute electrode portions 199a exposed to the outside. An electrode pair 207 shown in Fig. 14C is an example having a negative electrode 207b of a rod shape, instead of the negative electrode 187b of the flat plate shape in the electrode pair 197.

Electrode pairs 217, 227 shown in Figs. 14D and 14E are modification examples of the structure of the electrode pair 137. As in these examples, leading ends of two electrodes 217a, 217b in the electrode pair 217 may be configured to extend in opposite directions, or, base ends of two electrodes 227a, 227b in the electrode pair 227 may extend in proximity to each other. Furthermore, as in an electrode pair 237 shown in Fig. 14F, a leading end of a positive electrode 237a of a rod shape may be configured in a curved shape (e.g., an arcuate shape) along the bottom face of the well 21 and the leading end of the positive electrode 237a may be configured to face a leading end of a negative electrode 237b of a rod shape. The structures as described above can also efficiently apply the electrical stimulation in a large current density to a wide range of the sample S placed in the well 21.

In the above-mentioned cell observation apparatus, preferably, at least a part of a portion coated with the insulating material in the electrode pair has wettability. When this configuration is adopted, the solution can be efficiently drawn into the space between the first electrode and second electrode on the occasion of insertion of the electrode pair into the holding unit, and thus the reaction of the cell can be observed more accurately.

Preferably, the electrode pair is of such a cylindrical shape that the second electrode surrounds the first electrode. In this case, preferably, the first electrode is coated with the insulating material except for the leading end thereof facing the holding unit, and the second electrode is entirely exposed to the outside. When this configuration is adopted, the current density can be efficiently enhanced in the central part of the electrode pair and great electrical stimulation can be applied to the cell with low power consumption.

Preferably, the first and second electrodes are of a flat plate shape and are arranged so as to be parallel to each other. In this case, preferably, the electrical stimulation unit further has an insulator of a flat plate shape arranged between the first and second electrodes. When the foregoing configurations are adopted, the current density can be efficiently enhanced near the cell placed in the holding unit and great electrical stimulation can be applied to the whole cell with low power consumption.

Furthermore, preferably, the first and second electrodes are of a rod shape and are arranged so as to be parallel to each other. In this case, preferably, the electrode portion of the first electrode and the electrode portion of the second electrode constitute rod-shaped electrodes parallel to each other along a bottom face of the holding unit. This allows the electrical stimulation to be efficiently applied in a large current density to a wide range of the cell placed in the holding unit. In this case, the electrode portion of the second electrode may be of such a ring shape as to surround the electrode portion of the first electrode or may be of a curved shape facing the electrode portion of the first electrode. Such structures can efficiently apply the electrical stimulation in a large current density to a wide range of the cell placed in the holding unit.

Furthermore, preferably, the first electrode is of a rod shape, the second electrode is of a flat plate shape, and the first electrode is arranged along a surface of the second electrode. When this configuration is adopted, the electrical stimulation can be efficiently applied in a large current density to a wide range of the cell placed in the holding unit.

### Industrial Applicability

The present invention is used for the cell observation apparatus for observing the reaction of the sample including the cell to the electrical stimulation applied thereto and has enabled the improvement in accuracy of the cell observation with application of the electrical stimulation.

### Reference Signs List

1 cell observation apparatus; 11 microplate holder (mounting unit); 16 electrical stimulation unit; 17, 107, 117, 127, 137, 147, 157, 167, 177, 187, 197, 207, 217, 227, 237 electrode pairs; 17a, 107a, 117b, 137b, 147b, 157b, 167b, 177b, 187b, 207b, 217b, 227b, 237b negative electrodes (second electrodes); 17b, 117a, 137a, 147a, 157a, 167a, 177a, 187a, 197a, 217a, 227a, 237a positive electrodes (first electrodes); 117c insulating plate (insulators); 20 microplate (sample case); 21 wells (holding units); 22 bottom face; 71a, 71b, 118a, 118b, 138a, 138b insulators (insulating materials); 72a, 72b, 119a, 119b, 139a, 139b, 149a, 149b, 159a, 159b, 169a, 169b, 179a, 179b, 189a, 189b, 199a electrode portions; 73, 73A-73C spaces; L solution; S sample.

## Claims

1. A cell observation apparatus for observing a cell held by a sample case having an array of holding units holding a sample including the cell, the cell observation apparatus comprising:
a mounting unit for the sample case to be mounted thereon; and
an electrical stimulation unit in which a plurality of electrode pairs, each electrode pair including a first electrode and a second electrode, are arranged;
wherein at least one of the first electrode and the second electrode is coated with an insulating material except for a leading end thereof facing the holding unit, and the leading end forms an electrode portion exposed to the outside; and
wherein a space penetrating upward from the electrode portion is formed between the first electrode and the second electrode.

2. The cell observation apparatus according to claim 1,
wherein at least a part of a portion coated with the insulating material in the electrode pair has wettability.

3. The cell observation apparatus according to claim 1 or 2,
wherein the electrode pair is of such a cylindrical shape that the second electrode surrounds the first electrode.

4. The cell observation apparatus according to claim 3,
wherein the first electrode is coated with the insulating material except for the leading end thereof facing the holding unit, and
wherein the second electrode is entirely exposed to the outside.

5. The cell observation apparatus according to claim 1 or 2,
wherein the first and second electrodes are of a flat plate shape and are arranged so as to be parallel to each other.

6. The cell observation apparatus according to claim 5,
wherein the electrical stimulation unit further has an insulator of a flat plate shape arranged between the first and second electrodes.

7. The cell observation apparatus according to claim 1 or 2,
wherein the first and second electrodes are of a rod shape and are arranged so as to be parallel to each other.

8. The cell observation apparatus according to claim 7,
wherein the electrode portion of the first electrode and the electrode portion of the second electrode constitute rod-shaped electrodes parallel to each other along a bottom face of the holding unit.

9. The cell observation apparatus according to claim 7,
wherein the electrode portion of the second electrode is of such a ring shape as to surround the electrode portion of the first electrode.

10. The cell observation apparatus according to claim 7,
wherein the electrode portion of the second electrode is of a curved shape facing the electrode portion of the first electrode.

11. The cell observation apparatus according to claim 1 or 2,
wherein the first electrode is of a rod shape,
wherein the second electrode is of a flat plate shape, and
wherein the first electrode is arranged along a surface of the second electrode.
